# EUROPÄISCHE PATENTSCHRIFT

(11) **EP 1 458 414 B1**
(45) Veröffentlichungstag und Bekanntmachung des Hinweises auf die Patenterteilung: **14.04.2010**
(21) Anmeldenummer: 02804919.5
(22) Anmeldetag: 19.12.2002
(51) Int. Cl.: A61K 47/48, A61P 35/00

(54) **VERWENDUNG VON AN POLYALKYLENGLYCOL GEKOPPELTEN THROMBININHIBITOREN IN DER TUMORTHERAPHIE**
USE OF THROMBIN INHIBITORS COUPLED TO POLYALKYLENE GLYCOL IN TUMOUR THERAPY
UTILISATION DE INHIBITEURS DE LA THROMBINE COUPLES A DU POLYALKYLENE GLYCOL DANS LA THERAPIE DES TUMEURS

(30) Priorität: 19.12.2001 DE 10162521
(43) Veröffentlichungstag der Anmeldung: 22.09.2004
(73) Patentinhaber: Nowak, Götz, 99097 Erfurt (DE)
(72) Erfinder: NOWAK, Götz, 99097 Erfurt (DE); LOPEZ ZAMBRANO, Mercedes, 07747 Jena (DE)
(74) Vertreter: Vossius & Partner
(86) Internationale Anmeldenummer: PCT/EP2002/014590
(87) Internationale Veröffentlichungsnummer: WO 2003/051399

(56) Entgegenhaltungen:
- EP-A- 0 503 829
- WO-A-98/46648
- DE-A- 4 017 439
- DE-A- 10 102 878

## Beschreibung

Zahlreiche Tumortherapeutika entfalten ihre Wirkung an extrazellulären Rezeptoren von Tumorzellen und müssen damit langzeitig im extrazellulären Kompartment vorhanden sein. Um zu konstanten linearen Gewebespiegeln zu kommen, ist es von hohem Interesse, für die spezifische Verwendung solcher Wirksubstanzen Stoffe in ihrer chemischen Struktur so zu modifizieren, dass sie sich bevorzugt im extravasalen Teil des extrazellulären Wasserraums verteilen und nur geringe Konzentrationen im intravasalen Kompartment aufweisen. Dadurch wird sichergestellt, dass Blutungskomplikationen, die bei der langfristigen Verwendung von Hirudin oder kleinmolekularen synthetischen Thrombininhibitoren häufig auftreten, hier nicht entstehen können.

DE 4 017 439 A1 beschreibt tumoraktive oder Tumordiagnostik-Substanzen mit bevorzugter Anreicherung im Tumor und mit mindestens zwei phenolischen Hydroxylgruppen oder mindestens einer aliphatischen Aminogruppe, bei denen mindestens zwei phenolische Hydroxylgruppen oder mindestens eine aliphatische Aminogruppe mit einer Polyethylenglykolkette vom Polymerisationsgrad n = 5 bis 250 substituiert ist, deren endständige Hydroxylgruppe durch C₁-C₁₂-Alkylester oder -ether substituiert ist.

EP 0 503 829 A2 bezieht sich auf ein Verfahren zur Hemmung oder Vorbeugung von Krebsmetastasen mit Hilfe von Hirudin.

Die Aufgabe dieser Erfindung ist es, Wirkstoffe mit derartigen Eigenschaften, die bevorzugt für einen Einsatz in der Tumortherapie geeignet sind, bereit zu stellen. Sie sollen einen möglichst langzeitig konstanten extrazellulären Gewebespiegel erzeugen, ohne gleichzeitig möglicherweise toxische oder nebenwirkungsreiche hohe Blutspiegelwerte im Patienten zu präsentieren.

Diese Aufgabe wurde durch einen Thrombininhibitor gelöst, dessen Verteilungsgleichgewicht im Körper zugunsten des Extravasalraumes durch Kopplung an Polyalkylenglykol ohne eigene pharmakologische Wirkung verschoben ist. Polyalkylenglykole sind wasserlösliche PMV-Verbindungen (pharmacokinetic modulating vehicles) und als solche im Körper immunologisch und chemisch inert und besitzen keine biologische Wirkung. Thrombininhibitoren, deren Molekulargewicht mit Hilfe solcher sehr gut wasserlöslichen PMV erhöht wurde, zeichnen sich durch eine für die Tumortherapie besonders geeignete Pharmakokinetik aus. So wurde im Rahmen von Untersuchungen mit diesen Wirkstoffen überraschenderweise festgestellt, dass sich die molekulargewichtserweiterten Substanzen nur langsam im extrazellulären Wasserraum bewegen, denn der Austausch zwischen Extravasalraum und Intravasalraum erfolgt nur sehr verzögert. Von besonderem Interesse sind Verbindungen, die unterschiedliche Geschwindigkeitsparameter beim Ausstrom aus der Blutbahn und beim Wiedereintritt in die Blutbahn hatten. Beispielsweise verschwanden diese Substanzen mit relativ kurzen transkapillären Escape-Raten aus der Blutbahn, der Rücktransport aber durch die Endothellücken bzw. über andere Transportmechanismen aus dem Extravasalraum in den Blutraum erfolgte dann bedeutend langsamer.

Besonders geeignet als wasserlösliche Verbindungen (PMV) zur Einstellung des Verteilungsgleichgewichtes von Tumor-Therapeutika haben sich Polyalkylenglycole erwiesen. Es können einheitliche, aber auch unterschiedliche Glycole bei der Bildung des Polyalkylenglycols verwendet werden. Bevorzugt kommen C₁ - C₄ Polyalkylenglycole zum Einsatz, insbesondere Polyethylenglycol. Ihr Alkylenrest kann gerade oder verzweigt sein, und es können sowohl lineare als auch ring- oder sternförmige Polyalkylenglykole zum Einsatz kommen. Ihr Molekulargewicht liegt bevorzugt in einem Bereich von 0,5 bis 25 kDa. besonders bevorzugt ist eine Untergrenze des Molekulargewichts von 2 oder 2,5 kDa sowie Obergrenzen von 10 oder 5 kDa.

Der Polyalkylenglykol sollte durch eine chemisch stabile Bindung an das Wirkstoffmolekül gekoppelt werden, beispielsweise kovalent, wobei eine solche Bindung natürlich auch einen ionischen Anteil besitzen kann. Insbesondere eignen sich Ester oder Etherbindungen der endständigen OH-Gruppen mit entsprechenden funktionellen Gruppen des Wirkstoffmoleküls. Mehrfach funktionelle wasserlösliche Verbindungen können an ein, aber auch an mehrere Wirkstoffmoleküle gekoppelt sein (z.B. ein oder zwei Wirkstoffmoleküle im Falle eines linearen Polyalkylenglycols).

Durch die Molekulargewichtserweiterung mittels gut wasserlöslicher Substanzen wie dem Polyethylenglycol kann darüber hinaus die Pharmakokinetik der Wirksubstanzen so beeinflusst werden, dass sie nicht mehr durch die Leber verstoffwechselt, sondern überwiegend oder ausschließlich unverändert durch die Niere ausgeschieden werden. Polyalkylenglycole mit Molekulargewichten zwischen 2,5 und 10 kDa sind hier insofern bevorzugt, als sie eine verzögerte, aber noch nachweisbare renale Ausscheidung der modifizierten Wirksubstanzen ermöglichen.

Es ist seit langem bekannt, dass die Serinproteinase Thrombin, das zentrale Gerinnungsenzym, neben der unmittelbaren Blutgerinnungsauslösung und Blutplättchenaktivierung auch eine Reihe von zellulären, mitogenen Effekten hat. Diese mitogenen, zellwachstumsmodifizierenden Effekte des Thrombins werden über sogenannte PAR's (Protease-aktivierte Rezeptoren) an der Membranaußenseite von Zellen initiiert. Diese thrombinspezifischen Rezeptoren (PAR1, PAR3, PAR4) finden sich in ungewöhnlicher Häufigkeit an der Oberfläche von Tumorzellen. Dadurch können Serinproteinasen einen wachstumsmodifizieienden Einfluss auf Tumoren haben. Es ist bekannt, dass die Tumorinvasivität direkt korreliert mit der Zahl der PAR's an der Oberfläche der Tumorzellen. Aus Untersuchungen mit isolierten Zellen in den letzten 5 Jahren ist weiterhin bekannt, dass Thrombininhibitoren, die die Serinproteinaseaktivität effektiv blockieren, natürlich auch die mitogenen Effekte an den Zelloberflächen hemmen können. Damit ist mit Antithrombinen eine vollkommen neue zellwachstumsmodifizierende Wirkstoffgruppe vorhanden. Substanzen, die die Thrombinwirkung inhibieren, können als Cancerostatika dienen und eignen sich insbesondere zur Verwendung im Rahmen der vorliegenden Erfindung. Inhibitoren nach dem Prinzip der vorliegenden Erfindung gewährleisten dabei eine verbesserte Verfügbarkeit im extrazellulären Wasserraum, ohne dass dabei signifikante gerinnungsbeeinflussende Effekte oder toxische Substanzmengen im Blut auftreten können.

In der Substanzklasse der Antikoagulantien stehen zwei unterschiedliche Wirkstoffgruppen für die therapeutische Anwendung zur Verfügung: zum einen die natürlichen Thrombininhibitoren, wie Hirudin und Hirudinderivate als natürliche hochspezifische Thrombininhibitoren, zum anderen synthetische Thrombininhibitoren, z.B. Argatroban, Melagatran, NAPAP, CRC220 u.a., die in den letzten 10-15 Jahren in in vitro und in vivo Untersuchungen ihre thrombmspezifischen Hemmaktivitäten nachgewiesen haben. Die zielgerichtete Entwicklung von Thrombinhemmstoffen für einen zellulären Interaktionsmechanismus erfolgte bisher nicht. Bekannte Antithrombine sind ausschließlich für die Verwendung im zirkulierenden Blut vorgesehen, deshalb ist die Annahme, dass die Thrombin-antagonisierenden Wirkungen auch im extrazellulären Wasserraum verfügbar sind, nicht ohne weiteres möglich. Gerade für die spezifische Verwendung derartiger Thrombininhibitoren wäre es danach wünschenswert, sie in ihrer Struktur so zu modifizieren, dass sie sich bevorzugt im Extravasalraum verteilen und nur geringe Konzentrationen im intravasalen Kompartment aufweisen.

Als Leitsubstanz zur Antitumortherapie dient das Polyethylenglykol-gekoppelte Hirudin (PEG-Hirudin), ein Präparat, welches von der Knoll GmbH seit Anfang der 90er Jahre in präklinischen und klinischen Untersuchungen auf seine antithrombotische Wirksamkeit hin untersucht wurde. Die Pharmakokinetik des PEG-Hirudins unterscheidet sich grundsätzlich von der der Muttersubstanz, dem rekombinanten Hirudin (s. Tabelle 1)

**Tabelle 1; Pharmakokinetische Kenndaten von r-Hirudin und PEG-Hirudin**

| | r-Hirudin | PEG-Hirudin |
|---|---|---|
| t _{1/2α} (h) | 0,28 | 1,55 |
| t _{1/2β} (h) | 1,42 | 12,1 |
| Aus ₀₋ₜ (mg/l•h) | 2,89 | 23,8 |
| Cl ᵣₑₙ (ml/min) | 139,8 | 17,1 |
| V _{dss} (l/kg) | 0,251 | 0,248 |

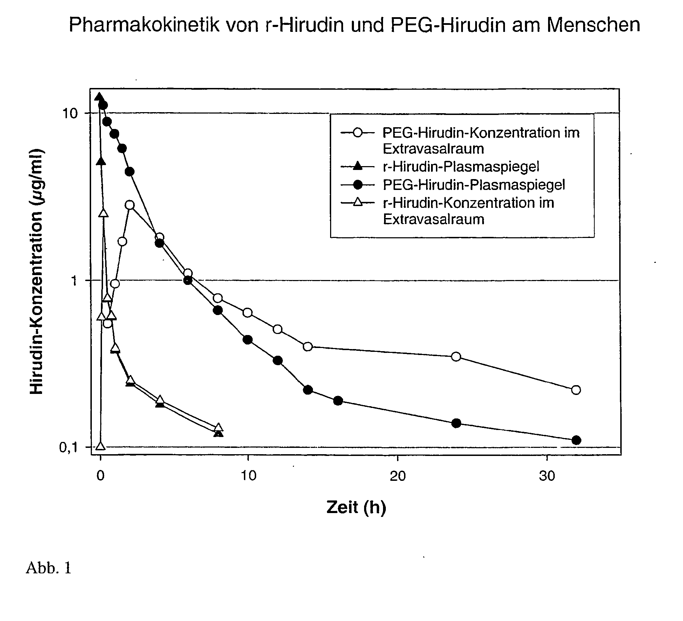

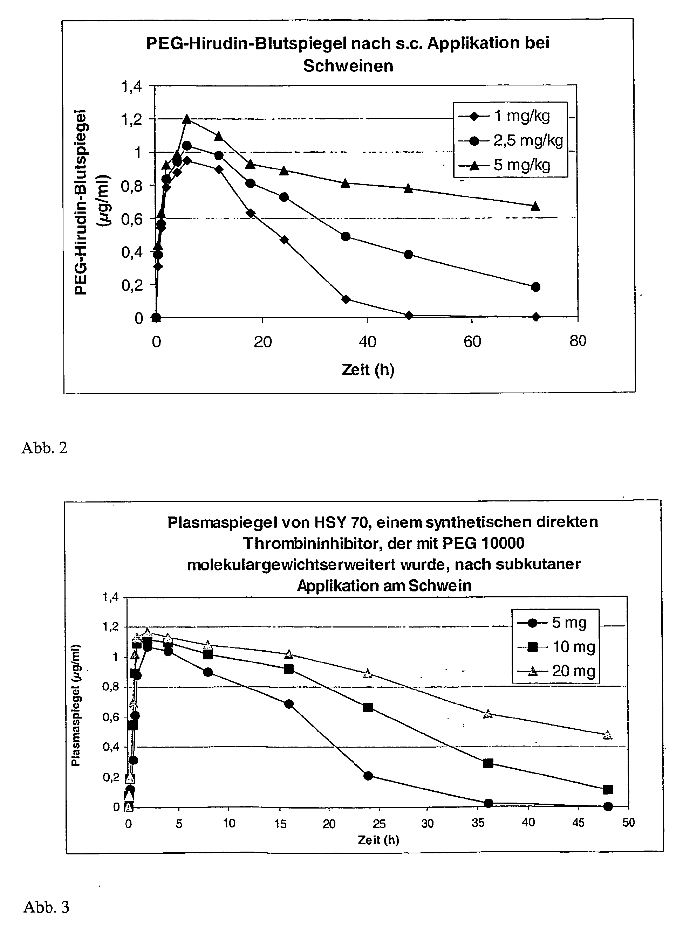

Rekombinantes Hirudin verteilt sich in wenigen Minuten in den extravasalen Raum und bildet bereits nach 20-30 min ein steady state zwischen dem Extravasal- und Intravasalraum. Das bedeutet, daß nach Applikation von r-Hirudin (die Substanz kann nur parenteral appliziert werden) ein sehr stark schwankender Blutspiegel und spiegelbildlich auch Gewebespiegel dieser Substanz zu finden sind, da aufgrund der rasch abfallenden Hirudin-Blutspiegelkurve und der ausschließlichen Elimination über die Nieren (Eliminationshalbwertzeit = 1-2 h) keine konstanten Gewebespiegel erzeugt werden können. (Abb. 1) Die einzige Möglichkeit zur Anwendung von r-Hirudin als Cancerostatikum bestünde also darin, diese Substanz per Dauerinfusion zu applizieren. Für eine chronische Anwendung über Tage, Wochen, Monate bzw. Jahre ist diese Technologie heutzutage nicht einsetzbar. Das Polyethylenglykol-gekoppelte Hirudin dagegen hat eine Eliminationshalbwertzeit von 8-10 h beim nierengesunden Patienten und einen sehr langsamen Austausch zwischen intravasalem und extravasalem Kompartment (Abb. 1). Die entsprechenden pharmakokinetischen Daten belegen, daß die Verteilungsphase bei dieser Substanz bereits 4-5 h lang ist. Erst nach dieser Zeit ist ein steady state zwischen intravasalem und extravasalem Kompartment vorhanden, so dass bei deutlich verzögerter Elimination des PEG-Hirudins über die Niere nur sehr langsam eine Konzentrationsabnahme im extrazellulären Kompartment stattfindet. Damit ist PEG-Hirudin ein Kandidat zur Verwendung als Antitumormittel. Nach subkutaner Applikation effektiver Dosen von PEG-Hirudin ist ein relativ konstantes Blutspiegelplateau von mehr als 24 h zu erreichen. Bei Dosiserhöhung ist nur eine sehr moderate Steigerung des Blutspiegels und damit auch Gewebespiegels zu erreichen. Die Erhöhung der Dosis führt jedoch zu einer zeitlichen Verlängerung des Blut-Gewebespiegel-Plateaus, so daß mit PEG-Hirudin bei strikter intrakutaner Verwendung (z.B. mit Hochdruckinjektoren, die eine sichere und exakte intrakutane Applikation zulassen) durchaus konstante Blut- und Gewebespiegelwerte bis zu drei Tagen erreicht werden (s. Abb. 2 ). Neben Hirudin können weitere natürliche Inhibitoren aus blutsaugenden Spezies zur Anwendung kommen, die durch die erfindungsgemäße Molekulargewichtserweiterung langzeitig konstante Blut- und Gewebespiegel nach einmaliger subkutaner Applikation erzeugen, wie sie für eine permanente Hemmung der Thrombinwirkung auf PAR-Rezeptoren von Bedeutung ist.

Neben natürlichen Wirkstoffen, ihren Derivaten, Mutanten oder Chimären eignen sich ebenfalls synthetische Thrombininhibitoren vom active site-gerichteten Typ. Direkte kleinmolekulare Thrombininhibitoren, die synthetisch hergestellt werden und eine ausreichend hohe Affinität zum Thrombin besitzen (Kᵢ-Werte < 1 nmol), sind in der Patentanmeldung Stüber et al.: EP 0 658 585 A2 beschrieben, aber auch in der deutschen Patentanmeldung von Steinmetzer et al. mit dem Aktenzeichen P10102878.4 dargestellt. Durch Kopplung mit einem PMV sind auch sie zur Anwendung im Rahmen der vorliegenden Erfindung geeignet. So erreichen beispielsweise mit Polyethylenglykol molekulargewichtserweiterte synthetische Thrombininhibitoren je nach Größe des PEG-Anteils ähnliche Langzeitkinetiken im Versuchstier wie PEG-gekoppelte natürliche Substanzen (s. Abb. 3). In Modelluntersuchungen am narkotisierten Schwein kann gezeigt werden, daß solche direkten synthetischen Antithrombine aufgrund der komplexen Molekrülstruktur ebenfalls nur parenteral appliziert werden können. Die Eliminationshalbwertzeiten, die bei pharmakokinetischen Untersuchungen am Schwein ermittelt wurden, liegen bei 12-15 h. Damit sind sehr lange konstante Plasma- und Gewebespiegel für diese Substanzen charakteristisch. Beispielsweise sind die synthetischen direkten Inhibitoren, deren Molekulargewicht nach dem Prinzip der vorliegenden Erfindung erhöht wurde, nach parenteraler Applikation, bevorzugt nach subkutaner Applikation, über mehr als 48 h sowohl im Blut als auch im extravasalen Wasserraum nachweisbar. Somit stellen molekulargewichtserweiterte synthetische Antithrombine ebenfalls wichtige potentielle Tumortherapeutika dar.

Ein weiterer substanzspezifischer Vorteil für die synthetischen. Thrombininhibitoren ist in der Tatsache zu sehen, daß diese Inhibitoren im Gegensatz zu Hirudin bzw. PEG-Hirudin kompetitive Inhibitoren und nicht slow tight-binding Inhibitoren sind. In Untersuchungen an Tumorzellmodellen konnte nämlich gezeigt werden, daß Konzentrationen von Thrombin < 1U/ml im Mikroenvironment die Tumorzellproliferation hemmen können, in höheren Konzentrationen das Thrombin jedoch die Tumorproliferation fördert. Umgesetzt auf in vivo Verhältnisse der Inhibitoren ist es damit von Vorteil, dass bei diesen kompetitiven Inhibitoren immer aufgrund ihrer Inhibitorkinetik ein gewisser Prozentsatz freier Inhibitor und freies Enzym vorliegt, so dass zusätzlich neben dem Blockieren der tumorproliferativen hohen Thrombinspiegel auch noch ein additiver thrombininhibitorischer Tumoreffekt auftreten kann. Alle bisher bekannt gewordenen synthetischen Thrombininhibitoren haben einen kompetitiven Hemmechanismus und sind aufgrund dieser Gegebenheiten weniger für die Thrombinhemmung in der Zirkulation bei thromboembolischen Erkrankungen als vielmehr als Tumor-inhibierende Substanzen zu favorisieren.

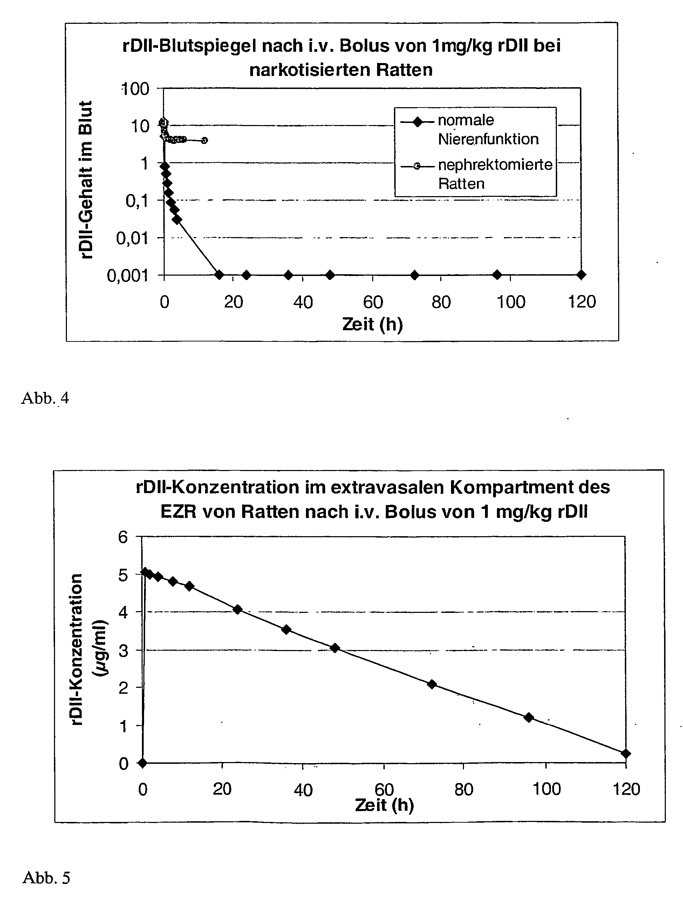

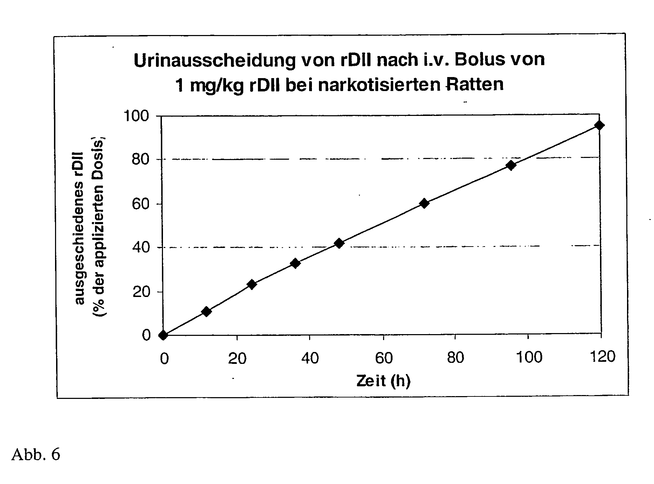

Eine weitere Gruppe von für die Indikation der Erfindung wirksamen Substanzen sind natürliche Thrombininhibitoren, die gentechnologisch hergestellt werden können und die bereits als solche eine ungleiche Verteilung zwischen Extravasal- und Intravasalraum aufweisen. Ein typischer Vertreter ist das r-Dipetalogastin, welches seit einiger Zeit in präklinischen Untersuchungen auf gerinnungshemmende und Thrombinrezeptorblockierende Wirkung hin untersucht wird. r-Dipetalogastin hat ein größeres Molekulargewicht als Hirudin (∼13 kDa) und zeigt wie letzteres einen sehr starken thrombininhibitorischen Effekt (Ki=130 fmol). Rekombinantes Dipetalogastin selbst, wie auch Mutanten und Chimeren dieses Thrombininhibitors, die mittels Gentechnik hergestellt werden, stimmen in ihrem pharmakokinetischen Verhalten präzise mit den gewünschten Langzeiteffekten im Extrazellulärraum überein und sind damit interessante Vertreter für Anti-Tumormittel. Erstmals konnte bei derartigen Substanzen gezeigt werden, dass sie eine ungleiche Verteilung i.S. einer schnellen Abnahme der Konzentration aus der Blutbahn und einem sehr langsamen Rückverteilen aus dem Extravasalraum in das Blutkompartment besitzen. Sie zeigen damit eine längere Verweildauer im extravasalen Kompartment und führen zu konstanten linearen Gewebespiegeln. Pharmakokinetisch verhalten sich diese Verbindungen genau wie Hirudin, sie werden ausschließlich über die Nieren eliminiert und haben mit dem Hirudin vergleichbare Kinetikdaten. Das r-Dipetalogastin ist in der Patentanmeldung Mende et al WO 00/24898 als neue rekombinante Antithrombinsubstanz dargestellt. Die Kopplung der dipetalogastinanalogen Substanzen mit PMV führt zu einer weiteren Optimierung ihres pharmakokinetischen Verhaltens. Es konnte z.B. an Polyethylenglycol-gekoppelten Substanzen gezeigt werden, dass sie nach einmaliger subkutaner Applikation bereits nach 10-15 h im Blut nicht mehr nachweisbar sind, jedoch länger als 5 Tage im Urin ausgeschieden werden. Diese Befunde, die bei pharmakokinetischen Untersuchungen an Versuchstieren erhoben wurden, lassen den Schluß zu, daß nur 10% der applizierten Substanz im Blut zu finden war, aber mehr als 90% dieser Dipetalogastin-analogen Substanzen im extrazellulären Raum verteilt wurden. Aufgrund der sehr verzögerten Rückverteilung bei first-pass-artiger guter Nierenausscheidung werden sie aus dem Blut entfernt. Dadurch werden konstante Wirkspiegel dieses Inhibitors im Extrazellulärraum erzeugt, ohne daß dabei in der Blutbahn nennenswerte gerinnungsbeeinflussende bzw. toxische Substanzmengen auftreten (s. Abb. 4-6).

Die molekulargewichtserweiterten Cancerostatika, insbesondere Thrombininhibitoren, stellen damit eine neue Arzneistoffgruppe zur Verwendung in der Tumortherapie zur Verfügung und sind Pilotsubstanzen für weitere Tumortherapeutika, die an extrazellulären Rezeptoren von Tumorzellen ihre Wirkung entfalten und langzeitig im extrazellulären Kompartment vorhanden sein müssen. Die Verwendung von beispielsweise PEGmolekulargewichtserweiterten Wirkstoffen zur Langzeittumortherapie ist damit von großer Bedeutung für zukünftige, im extrazellulären Raum wirksame Arzneimittel geworden.

## Patentansprüche

1. Thrombininhibitor, gekoppelt an Polyalkylenglycol zur Behandlung von Tumorerkrankungen.

2. Thrombininhibitor zur Behandlung nach Anspruch 1, wobei es sich bei dem Polyalkylenglycol um ein Polyethylenglycol handelt

3. Thrombininhibitor zur Behandlung nach Anspruch 1 oder 2, wobei es sich um einen natürlichen oder synthetischen Thrombininhibitor handelt.

4. Thrombininhibitor zur Behandlung nach einem der Ausprüche 1 bis 3,
wobei es sich bei dem Inhibitor um Hirudin oder Dipetalogastin handelt.

5. Thrombininhibitor zur Behandlung nach einem der Ausprüche 1 bis 3, wobei es sich bei dem Inhibitor um Argatroban, Melagatran, NAPAP oder CRC220 handelt.

## Claims

1. A thrombin inhibitor, coupled to polyalkylene glycol, for the treatment of tumour diseases.

2. The thrombin inhibitor for treatment according to claim 1, wherein the polyalkylene glycol is a polyethylene glycol.

3. The thrombin inhibitor for treatment according to claim 1 or 2 being a natural or synthetic thrombin inhibitor.

4. The thrombin inhibitor for treatment according to any one of claims 1 to 3, wherein the inhibitor is Hirudin or Dipetalogastin.

5. The thrombin inhibitor for treatment according to any one of claims 1 to 3, wherein the inhibitor is Argatroban, Melagatran, NAPAP or CRC220.

## Revendications

1. Inhibiteur de la thrombine couplé à un polyalkylène glycol pour le traitement de maladies tumorales.

2. Inhibiteur de la thrombine pour le traitement selon la revendication 1, dans lequel le polyalkylène glycol est un polyéthylène glycol.

3. Inhibiteur de la thrombine pour le traitement selon la revendication 1 ou la revendication 2, dans lequel l'inhibiteur de la thrombine est un inhibiteur naturel ou synthétique.

4. Inhibiteur de la thrombine pour le traitement selon l'une des revendications 1 à 3, dans lequel l'inhibiteur est l'hirudine ou la dipétalogastine.

5. Inhibiteur de la thrombine pour le traitement selon l'une des revendications 1 à 3, dans lequel l'inhibiteur est l'argatroban, le mélagatran, le NAPAP ou le CRC220.
